# EUROPÄISCHE PATENTSCHRIFT

(11) **EP 1 801 114 B1**
(45) Veröffentlichungstag und Bekanntmachung des Hinweises auf die Patenterteilung: **16.09.2009**
(21) Anmeldenummer: 05025499.4
(22) Anmeldetag: 23.11.2005
(51) Int. Cl.: C07H 21/00

(54) **Polynukleotid mit Phosphatmimetikum**
Polynucleotides with a phosphate mimetic
Polynucléotides contenant des phosphates mimétiques

(43) Veröffentlichungstag der Anmeldung: 27.06.2007
(73) Patentinhaber: Roche Diagnostics GmbH, 68305 Mannheim (DE); F.HOFFMANN-LA ROCHE AG, 4070 Basel (CH)
(72) Erfinder: Heindl, Dieter, 82396 Paehl (DE); Kessler, Dirk, 86971 Peiting (DE)

(56) Entgegenhaltungen:
- WO-A-01/14401
- JIN Y ET AL: "Parallel solid-phase synthesis of nucleoside phosphoramidate libraries" BIOORGANIC & MEDICINAL CHEMISTRY LETTERS, OXFORD, GB, Bd. 11, 2001, Seiten 2057-2060, XP002221668 ISSN: 0960-894X
- BASCHANG G ET AL: "IMIDOPHOSPHATE ALS NEUE NUCLEOTID-DERIVATE" ANGEWANDTE CHEMIE, VCH VERLAGSGESELLSCHAFT, WEINHEIM, DE, Bd. 85, Nr. 1, 1973, Seiten 43-44, XP000944429 ISSN: 0044-8249
- JOHN NIELSEN, MARVIN H. CARUTHERS: "Directed Arbuzov-type Reactions of 2-Cyano-1,1-dimethylethyl-Deoxynucleoside Phosphites" JOURNAL OF THE AMERICAN CHEMICAL SOCIETY, Bd. 110, 1988, Seiten 6275-6276, XP002377465

## Beschreibung

Die vorliegende Erfindung betrifft neue Stoffe und Verfahren zu deren Herstellung auf dem Gebiet der Nukleotid Chemie. Dabei handelt es sich um so genannte Phosphatmimetika, bei denen eine Hydroxylguppe durch ein entsprechendes Mimetikum ersetzt ist.

Insbesondere betrifft die vorliegende Erfindung eine neue Klasse von modifizierten Oligonukleotiden sowie Verfahren zu deren Herstellung.

### Stand der Technik

In der Vergangenheit wurden bereits verschiedene Verfahren zur Herstellung von am Phosphatrest modifizierten Nukleotiden oder Oligonukleotiden beschrieben.

Die Herstellung synthetischer (Desoxy-)-Oligonukleotide erfolgt in der Regel an einer Festphase mit Hilfe der Phosphoramidit-Chemie. Als Festphase werden üblicherweise Glasbeads mit Poren definierter Größe eingesetzt (im Folgenden mit CPG = controlled pore glass abgekürzt). Das erste Monomer ist über eine abspaltbare Gruppe mit dem Träger verbunden, so dass nach Beendigung der Festphasensynthese das freie Oligonukleotid abgespalten werden kann. Zusätzlich enthält das erste Monomer eine geschützte Hydroxylgruppe, wobei üblicherweise als Schutzgruppe Dimethoxytrityl (DMT) verwendet wird. Die Schutzgruppe kann durch Säurebehandlung entfernt werden. In einem zyklischen Verfahren werden dann nacheinander am 5'-Ende ebenfalls mit einer DMT-Schutzgruppe versehene 3'-Phosphoramidit-Derivate von (Desoxy-)-Ribonukleosiden an die jeweils von der DMT-Schutzgruppe befreite reaktive Gruppe gekoppelt. Alternativ werden in der inversen Oligonukleotidesynthese 3' Dimethoxytrityl geschützte 5' Phosphoramidte eingesetzt. Insbesondere zum Einführen von Modifikationen am Phosphatbackbone, z.B. zur Herstellung radiogelabelter Phosphorthioaten, wird auch die H-Phosphonatstrategie eingesetzt. Verschiedene Strategien sind auch bereits bzgl der Herstellung von modifizierten bzw. markierten Oligonuleotiden bekannt: Für die Herstellung von am 3'-Ende-markierten Oligonukleotiden werden gemäß dem Stand der Technik trifunktionelle Trägermaterialien verwendet (US 5,290,925, 5,401,837). Zur Herstellung von am 5'-Ende markierten Oligonukleotiden werden üblicherweise mit einer Markierung versehene Phosphoramidite verwendet, bei denen die Markierungsgruppe über einen C3-12 Linker mit dem Phosphoramidit verbunden ist (US 4,997,928, US 5,231,191). Darüber hinaus können Modifikationen an Oligonukleotiden an den einzelnen Basen (US 5,241,060, US 5,260,433, US 5,668,266) oder durch Einführung von internen Nicht-Nukleosidlinkern (US 5,656,744, US 6,130,323) eingeführt werden.

Alternativ kann eine Markierung am Internukleosid-Phosphat durch postsynthetische Markierung von Phosphorthioaten (Hodges, R.R., et al., Biochemistry 28 (1989) 261-7) oder durch Postlabeling eines funktionalisierten Phosphoramidates durchgeführt werden (S. Agrawal In Methods in Mol. Biology Vol 26 Protocols for Oligonucleotide Conjugates Humana Press, Totowa, NJ, 1993, Chapter 3). Diese Verfahren haben sich jedoch aufgrund der Instabilität der Phosphoramidate und Phosphorsäurethioester nicht durchgesetzt.

Auch die Einführung von Modifikationen am Inter-Nukleosid-Phosphatrest von Oligonukleotiden ist bereits aus dem Stand der Technik bekannt. Dabei handelt es sich in den prominentesten Fällen um Phosphothioate (Burgers, Peter M.J., und Eckstein, Fritz, Biochemistry 18, (1979) 592-6), Methyl-Phosphonate (Miller, P.S., et al., Biochemistry 18 (1979) 5134-43) oder Borano-Phosphate (WO 91/08213). Zur Herstellung von Methylphosphonat Oligonukleotiden müssen spezielle Monomere synthetisiert werden. Hingegen können zur Synthese von Phosphorthioaten und Boranophosphaten konventionelle Phosphoramidite oder H-Phosphonate verwendet werden, wobei die Borano- oder Thio Modifikation durch Verwendung spezieller Reagenzien, die mit dem dreiwertigen H- Phosphonat bzw. mit dem Phosphonsäuretriester reagieren, direkt während oder auch nach der Oligonukleotidsynthese eingeführt werden. Obwohl all diese Verfahren zu modifizierten Oligonukleotiden führen, erlauben die Erfordernisse der dafür angewandten Synthesechemie jedoch nicht, auf diese Weise detektierbare Markierungen oder auch funktionelle Gruppen am Phosphatbackbone der Oligonukleotidkette direkt während der Oligonukleotidsynthese einzuführen.

Baschang, G., und Kvita, V., Angewandte Chemie 85(1) (1973) 43-44 beschreiben die Umsetzung eines Nukleotid-Phosphorsäure-Tri-Esters mit Aziden wie bspw. Methyl-Sulfonyl-Azid zur Herstellung von Tri-alkyl(aryl)-Imidophosphaten, die allerdings instabil sind und zerfallen.

Nielsen, J., und Caruthers, M.H., J. Am. Chem. Soc. 110 (1988) 6275-6276 beschreiben die Umsetzung von mit einer 2-Cyano-1,1,-dimethylethyl Schutzgruppe versehenen Deoxynucleosid-Phosphiten in Gegenwart von Alkyl-Azid. Darüber hinaus schlagen die Autoren vor, dass dieses Prinzip zur Herstellung von am Phosphatrest modifizierten Nukleotiden geeignet ist, ohne darauf einzugehen, welche Arten von mithilfe des offenbarten Verfahren hergestellten Modifikationen besondere Vorteile aufweisen könnten. Insbesondere schlagen die Autoren die Einführung von Alkylresten vor.

WO 89/091221 offenbart N-Alkyl-Phosphoramidate, bzw. an mindestens einem Phosphatrest mit N-Alkyl-substituierte Oligonukleotide, die durch Oxidation von (mit einer Schutzgruppe versehen) Nukleosid-Phosphiten mit Jod in Gegenwart von geeigneten Alkylaminen hergestellt werden.

WO 03/02587 offenbart die Herstellung von modifizierten Oligonukleotiden, bei denen H-Phosphate durch Aminierung zu Phosphor-Amidaten umgesetzt werden.

Somit beschreiben all diese Publikationen die Herstellung von Molekülen, die anstelle eines Phosphatrestes ein Phosphoramidat enthalten. Phosphoramidat enhaltende Moleküle sind jedoch hydrolyselabil, da die Amingruppe im sauren Milieu protoniert wird und dann durch Wasser substituiert wird.

Darüber hinaus schlägt WO 01/14401 Nukleotidbausteine bzw. Oligonukleotide vor, bei denen ein Phosphatrest mit N--ClO3, N--NO2 oder N--SO2R substituiert ist. Gemäß der Lehre aus WO 01/14401 können derartige Substanzen dadurch hergestellt werden, dass die freie Hydroxylgruppe eines Deoxy-Nukleosids in Gegenwart von Pyridin mit Amidophosphonylchlorid umgesetzt wird. Diese Art der Herstellung ist allerdings umständlich, zeitaufwendig und für Routinesynthesen von Nukleotiden bzw Oligonukleotiden ungeeignet.

Die der vorliegenden Erfindung zugrunde liegende technische Aufgabe bestand damit in der Bereitstellung von verbesserten markierten Oligonukleotiden sowie in der Bereitstellung von einfachen Verfahren zu deren Herstellung.

### Kurzbeschreibung der Erfindung

Gegenstand der vorliegenden Erfindung ist daher ein Oligonukleotid, enthaltend mindestens einmal die Struktur 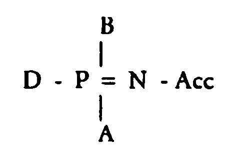 wobei A das 5' Ende eines Nukleotids oder einer Nukleotidkette darstellt, und
B das 3' Ende eines Nukleotids oder einer Nukleotidkette darstellt,
D entweder Hydroxyl oder CH3 ist und
Acc ein Elektronenakzeptor oder ein mit einem Rest R substituierter Elektronenakzeptor ist und R ein beliebiger organischer Substituent ist, und
Acc ausgewählt wird aus einer Gruppe bestehend aus
- -CN,
- -SO2 - R', wobei R' mindestens ein Amino-substituiertes Alkyl, ein gegebenenfalls substituiertes Aryl oder einen gegebenenfalls substituierten Heterozyklus enthält,
- und sechsgliedrigen N+ - Heterozyklen, ausgewählt aus einer Gruppe bestehend aus Pyridinium, Pyrimidinium und Chinolinium.

Besonders bevorzugt sind Oligonukleotide, bei denen R oder R' alleine oder in Kombination mit der Elektronenakzeptor eine detektierbare Einheit oder eine funktionelle Gruppe enthalten.

Erfindungsgemäß werden diese Oligonukleotide durch Verfahren hergestellt, welche
dadurch gekennzeichnet sind, dass ein 3-wertiges Phosphorderivat der chemischen Struktur 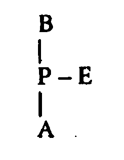 wobei E entweder eine Methylgruppe oder eine geschützte Hydroxylgruppe darstellt,
wobei A das 5' Ende eines Nukleotids oder einer Nukleotidkette darstellt, und
wobei B das 3' Ende eines Nukleotids oder einer Nukleotidkette darstellt,
umgesetzt wird mit einem Azid der Struktur

N=N=N-Acc

wobei Acc ein Elektronenakzeptor oder ein mit einem Rest R substituierter Elektronenakzeptor ist und R ein beliebiger organischer Substituent ist.

Der Elektronenakzeptor Acc wird ausgewählt aus einer Gruppe bestehend aus
- CN, - SO2 -R
- und sechsgliedrigen N+ - Heterozyklen, die ausgewählt sind aus einer Gruppe bestehend aus Pyridinium, Pyrimidinium und Chinolinium.

In einer besonderen Ausführungsform zur Herstellung eines erfindungsgemäßen Oligonukleotids erfolgt zunächst
a) die Umsetzung eines 3' Phosphoramidits mit dem 5' -OH Ende einer naszierenden Oligonukleotidkette
   und anschließend
b) die Umsetzung mit einem Azid der Struktur

   N=N=N-Acc,

   wobei Acc ein Elektronenakzeptor oder ein mit einem Rest R substituierter Elektronenakzeptor ist und R ein beliebiger organischer Substituent ist.

Besonders bevorzugt sind Verfahren, bei denen R eine detektierbare Einheit oder eine funktionelle Gruppe enthalten.

Die auf diese Weise hergestellten erfindungsgemäßen Oligonukleotide können für alle Anwendungen eingesetzt werden, in denen in jeglicher Form Hybridisations-Partner und insbesondere derivatisierte oder markierte Hybridsationspartner erforderlich sind.

Insbesondere können diese Oligonukleotide als Hybridisationssonden zur Detektion von bestimmten Target-Sequenzen verwendet werden.

Eine weitere potentielle Verwendung betrifft den Einsatz von erfindungsgemäß modifizierten Oligonukleotiden zur Inaktivierung von Genexpresssion im Sinne von Antisense Oligonukleotiden oder siRNAs.

### Detaillierte Beschreibung der Erfindung

### Grundgedanke der Erfindung

Ziel der vorliegenden Erfindung ist es, Nukleotide bzw. Oligonukleotide auf einfache Weise herzustellen, die modifizierte Phosphatreste enthalten und somit vorzugsweise auch detektierbare Markierungen enthalten können.

Kerngedanke der vorliegenden Erfindung war es in diesem Zusammenhang, von einem dreiwertigen Phosphoratom auszugehen und dieses mit einem Reagenz so umzusetzen, dass ein stabiles Phosphatmimetikum entsteht. Erfindungsgemäß wird dazu ein Phosphoratom, enthaltend mindestens einen Hydroxylrest, welcher mit einer Schutzgruppe versehenen ist, umgesetzt mit einem Azid der Struktur N = N = N - Acc, wobei Acc ein Elektronenakzeptor oder ein mit einem Rest R substituierter Elektronenakzeptor ist und R ein beliebiger organischer Substituent ist. Dadurch entseht ein 5-wertiges Phosphoratom , an das über ein N-Atom kovalent an eine starkelektronenziehende Elektronenenakzeptorgruppe gebunden ist. Diese Gruppe sorgt dafür, dass die auf diese Weise hergestellten Verbindungen im Gegensatz zu den aus dem Stand der Technik bekannten Phosphoramidat-Verbindungen mesomeriestabilisert und nicht hydrolyse-labil sind.

Dieser der der Erfindung zugrunde liegende Gedanke lässt sich auf alle Verfahren übertragen bei denen ein dreiwertiger Phosphor als Intermediat entsteht.

Während einer konventionellen Oligonukleotidsynthese unter Verwendung von Phosphor-Amiditen entstehen als Zwischenprodukte Phosphonsäure-Tri-ester mit einem 3-wertigen Phosphoratom. Die erste und zweite Esterbindung stellen die Internukleosidverknüpfung dar. Über die dritte Esterbindung ist das Phosphoratom mit einer geschützten Hydroxylgruppe wie bspw. mit einer beta-Cyanoethyl-oxygruppe verbunden. Anstelle einer Oxidation mit Jod kann dann das naszierende Oligonukleotid erfindungsgemäß mit einem entsprechenden Azid umgesetzt werden, wobei das dreiwertige Phosphoratom zu einem 5-wertigen Atom oxidiert wird, indem unter Abspaltung von Stickstoff -N-Acc kovalent mit dem Phosphoratom verknüpft wird.

Anschließend kann die Oligonukleotidsynthese wie aus dem Stand der Technik bekannt fortgesetzt werden. Als Endprodukt werden stabile Oligonukleotide erhalten, die an die an einem oder mehreren inter-Nukleotid-Phosphatresten auf nahezu beliebige Art und Weise modifiziert sind.

### Definitionen

Im Rahmen der vorliegenden Erfindung sind einige der verwendeten Begriffe wie folgt zu definieren:

Als reaktive Gruppe werden Gruppen eines Moleküls bezeichnet, die in der Lage sind, bei geeigneten Bedingungen unter Entstehung einer kovalenten Bindung mit einem weiteren Molekül zu reagieren. Beispiele für reaktive Gruppen sind Hydroxyl-Gruppen, Amino-Gruppen Thiol-, Hydrazino-, Hydroxylamino-, Diene, Alkine und Carbonsäure-Gruppen.

Als Schutzgruppe werden Moleküle bezeichnet, die mit einer oder mehreren reaktiven Gruppen eines Moleküls reagieren, sodass im Rahmen einer mehrstufigen Synthese-Reaktion nur eine bestimmte, nicht geschützte reaktive Gruppe mit dem gewünschten Reaktionspartner reagieren kann. Beispiele für häufig verwendete Schutzgruppen zum Schutz von Hydroxlgruppen sind beta-Cyano-Ehyl, - Methyl, Trialkylsilyl- und Allyl-. Schutzgruppen zum Schützen von Aminogruppen sind Trifluoracetyl und Fmoc. Weitere mögliche Schutzgruppen sind in Standardwerken zusammengefasst. (Greene T.W. Protective groups in organic synthesis.Wiley Interscience Publications John Wiley&Sons, 1981 New York, Chichester, Brisbane Toronto; Souveaux, E., Methods in Mol. Biology, Vol 26, Protocols for Oligonucleotide Conjugates, Humana Press, Totowa, NJ, 1994, Chapter 1, ed. S. Agrawal).

Als Linker werden Kohlenstoffketten mit einer Länge von 1-30 C-Atomen bezeichnet. Derartige Linkerketten können außerdem auch noch einzelne oder mehrere Stickstoff- Sauerstoff-, Schwefel- und/oder Phosphoratome aufweisen. Linker können darüber hinaus auch verzweigt z.b. auch dendritisch sein

Unter einer detektierbaren Einheit sind Substanzen zu verstehen, die mit Hilfe von analytischen Methoden nachgewiesen werden können. Dabei kann es sich beispielsweise um massenspektroskopisch, immunologisch,oder mit Hilfe von NMR nachweisbare Einheiten Substanzen handeln. Insbesondere sind unter detektierbaren Einheiten auch durch optische Methoden wie Fluoreszenz und UV/VIS-spektroskopie nachweisbare Substanzen wie beispielsweise Fluoresceine, Rhodamine oder auch Goldpartikel zu verstehen. Dazu gehören auch Interkalatoren und Minor groove Binder, die gleichzeitig einen Einfluß auf das Schmelzverhalten haben und ihre Fluoreszenz durch Hybridisierung ändern.

Als Phosphoramidite werden Moleküle mit einem dreiwertigen Phosphor-Atom bezeichnet, die an das 5'-terminale Ende eines Nukleosids bzw. Nukleosidderivates gekoppelt werden können. Somit sind Phosphoramidite bei der Oligonukleotidsynthese einsetzbar. Neben den für eine Kettenverlängerung verwendeten (Desoxy)-Ribonukleotid-Phosphoramiditen existieren mit einer Markierung derivatisierte Phosphoramidite, die in analogen Verfahren während bzw. am Ende der Oligonukleotidsynthese zur Markierung des Oligonukleotids verwendet werden können (Beaucage, S.L., Methods in Molecular Biology 20 (1993) 33-61, ed. S. Agrawal, Wojczewski, C., et al., Synlett 10 (1999) 1667-1678).

Der Begriff "Oligonukleotide" subsummiert im Zusammenhang mit der vorliegenden Erfindung nicht nur (Desoxy)-Oligo-Ribonukleotide, sondern auch Oligonukleotide, die ein oder mehrere Nukleotid-Analoga mit Modifikationen am Phosphat-Backbone (wie beispielsweise Methylphosphonate, Phosphothioate), am Zucker (wie z.B. oder 2'-O-Alkylderivate 3' und/ oder 5' Aminoribose, LNA, HNA, TCA) oder modifizierte Basen wie z.Bsp. 7-Deazapurine enthalten. Bestandteil der Erfindung sind in diesem Zusammenhang auch Konjugate und Chimäre mit nicht nukleosidischen Analogas wie z.B. PNAs oder anderen Biopolymeren wie z.B. Peptiden. Darüber hinaus können die erfindungsgemäßen Oligonukleotide auch an jeder Position einzelne oder mehrere nicht-nucleosidische Einheiten wie Spacer, bspw. Hexaethylenylcol oder Cn (n= 3,6)-Spacer enthalten.

Der Begriff "Elektronenakzeptor" umfasst atomare Struktur, welche die Tendenz besitzen, freie Elektronenpaare an sich zu binden. Ein Maß dafür ist die HammetKonstante. Gegenstand der vorliegenden Erfindung sind insbesondere Ausführungsformen, bei denen die Hammetkonstante σₚ () einen bestimmten Wert von 0.30, vorzugsweise 0.45, und besonders bevorzugt 0.60. überschreitet.

Zusätzlich muß der Elektronenakzeptor kompatibel mit allen chemischen reaktionen im Rahmen der Oligounkleotidsynthese sein, d.h.
- er darf nicht oxidierbar durch Iod sein
- er muß inert gegenüber Dichloressigsäure und Trichloressigsäure sein, und
- er muß inert gegenüber Basen wie insbesondere Ammoniak sein, und
- er darf nicht mit dreiwertigem Phosphoramidaten reagieren.

Beispiele für Elektronenakzeptoren, welche diese Bedingungen erfüllen sind:
- NO2, -SO2-R, -CN, -CO-R, Pyrinidinyl-, Pyridinyl-, Pyridazinyl-, Hexafluorophenyl-, Benzo-triazolyl-, e.c. (Hansch, C., et al., Chem Reviews 91 (1991) 165-195). Darüber hinaus können diese Akzpetoren auch vinylog oder phenylog an das Stickstoffatom gebunden sein.

Der Begriff "substituiert ...." bedeutet, dass die jeweils als substituiert bezeichnete Struktur einen weiteren Rest an einer beliebigen Position enthält, sofern diese Position nicht näher definiert ist. Der Begriff "gegebenenfalls substituiert" oder "optional substituiert" bedeutet, dass die so bezeichnete Struktur Ausführungsformen mit und ohne zusätzlichen Rest umfasst.

Der Begriff ""Amino-substituiertes Alkyl" umfasst C₁-C₃₀ linear oder verzweigtes Alkyl, dass mindestens eine Aminogruppe enthält, wobei diese Aminogruppe geschützt ist oder über einen Linker mit einer detektierbaren Einheit verbunden ist

Der Begriff "elektronenarmer, sechsgliedriger N+-Heterozyklus" beinhaltet N-Heterozyklen, die an einem sp2 Stickstoff alkyliert sind, so dass die Ladung des Heterozyklus insgesamt positiv ist. Beispiele dafür sind Pyridinium, Pyrimidinium und Chinolinium.

### Erfindungsgemäße Oligonukleotide

Die vorliegende Erfindung umfasst jegliches Oligonukleotid, enthaltend mindestens einmal die Struktur 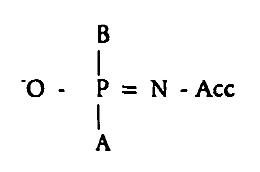 wobei
A das 5' Ende eines Nukleotids oder einer Nukleotidkette darstellt, und
B das 3' Ende eines Nukleotids oder einer Nukleotidkette darstellt und Acc ein Elektronenakzeptor oder ein mit einem Rest R substituierter Elektronenakzeptor ist und R ein beliebiger organischer Substituent ist. Dabei muß dieser Rest Zusätzlich muß der kompatibel mit allen chemischen Reaktionen im Rahmen der Oligounkleotidsynthese sein, d.h.
- er darf nicht oxidierbar durch Iod sein
- er muß inert gegenüber Dichloressigsäure und Trichloressigsäure sein, und
- er muß inert gegenüber Basen wie insbesondere Ammoniak sein, und
- er darf nicht mit dreiwertigem Phosphoramidaten reagieren.

Zunächst per se nicht kompatible Reste können allerdings durch Verwendung von dem Fachmann bekannten Schutzgruppen in Derivate umgewandelt werden, welche sich unter den chemischen Bedingungen der Oligonukleotidsynthese inert verhalten.

Darüber hinaus beinhaltet die vorliegende Erfindung auch Methyl-Phosphonate der Struktur

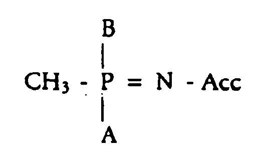

Abhängig von der beabsichtigten Verwendung des Oligonukleotids können die oben beschriebenen Strukturen einmal, zweimal, vielfach oder sogar an allen im Oligonukleotid vorhandenen Phosphatresten vorkommen. Innerhalb des Oligonukleotids handelt es sich bei den Phosphatresten um so genannte Internukleosidphosphate, wobei
- A: das 5'-Ende eines ersten Nukleosids und
- B: das 3'-Ende eines zweiten Nukleosids innerhalb der Nukleotidkette darstellt.

Darüber hinaus können die erfindungsgemäßen Strukturen sich am 3'- oder 5'-Ende eines Oligonukleotids befinden. Im Falle des Vorhandenseins am 5'-Ende des Oligonukleotids stellt
- A: das 5'-Ende der Nukleotidkette dar und
- B: ist entweder eine Hydroxylgruppe oder ein Linker, der optional eine detektierbare Gruppe oder eine weitere reaktive Gruppe enthalten kann, über den eine detektierbare Gruppe an das Oligonukleotid synthetisiert werden kann.

Enthält der Elektronenakzeptor einen Substituenten, welcher ebenfalls eine detektierbare Einheit darstellt, so liegt erfindungsgemäß ein Oligonukleotid mit einer dualen Markierung am 5'-Ende vor. Befindet sich die erfindungsgemäße Struktur am 3'-Ende einer Nukleotidkette, so repräsentiert
B das 3'-Ende des besagten Oligonukleotids und
A ist entweder Hydroxyl oder ein an eine Festphase gebundener Linker, wobei es sich bei der Festphase vorzugsweise um Controlled Pore Glass-particles handelt, wie sie als Ausgangsmaterial für die routinemäßige Oligonukleotidsynthese verwendet werden.

Die einzelnen Nukleoside innerhalb der erfindungsgemäßen Oligonukleotide können jegliche Art von Nukleosiden bzw. modifizierten Nukleosiden oder Nukleosidderivaten enthalten. Bei den Zuckereinheiten handelt es sich in der Regel um Deoxyribose für DNA-Oligonukleotide bzw. Ribose für RNA-Oligonukleotide. Die in den erfindungsgemäßen Oligonukleotiden enthaltenen Nukleobasen können natürlich vorkommende Basen wie Adenin, Guanin, Thymidin, Cytidin, Uridin , deren Derivate oder so genannten Universal Bases, wie beispielsweise Nitroindol, darstellen. Die erfindungsgemäßen Oligonukleotide enthalten Elektronenakzeptorgruppen, welche über eine Amidbindung mit dem jeweiligen Phosphat verknüpft sind. Insbesondere können folgende Elektronenakzeptorgruppen verwendet werden:
a) - CN
b) - SO2 - R', wobei R' mindestens ein Amino-substituiertes Alkyl, ein gegebenenfalls substituiertes Aryl oder einen gegebenenfalls substituierten Heterozyklus enthält,
c) sechsgliedrigen N+ - Heterozyklen, ausgewählt aus einer Gruppe bestehend aus Pyridinium, Pyrimidinium und Chinolinium.

Bestandteil der Erfindung sind dabei zweifelsfrei auch Ausführungsformen von - SO2 - R', bei denen R' als solches ein Amino-substituiertes Alkyl, ein gegebenenfalls substituiertes Aryl oder einen gegebenenfalls substituierten Heterozyklus ist.

Die Präsenz aller genannten Elektronenakzeptoren innerhalb der erfindungsgemäßen Oligonukleotide führt zu modifizierten Oligonukleotiden, welche für verschiedenste Anwendungen Verwendung finden können. Von besonderem Interesse sind jedoch sämtliche Elektronenakzeptoren, die einen beliebigen organischen Rest R enthalten können, weil die im Rahmen dieser Anmeldung beschriebenen Syntheseverfahren somit die einfache Herstellung von mit beliebigen organischen Resten modifizierten Oligonukleotiden ermöglichen.

Gegenstand der vorliegenden Erfindung sind somit insbesondere auch Oligonukleotide, bei denen ein mit einem Rest R substituierter Elektronenakzeptor als R eine detektierbare Einheit enthält oder alternativ eine funktionelle Gruppe enthält, an die imAnschluß an die Oligonukleotidsynthese durch "Postlabeling" eine detektierbare Einheit gekoppelt werden kann. Alternativ umfasst die vorliegende Erfindung auch Ausführungsformen, bei denen der Elektronenakzeptor Bestandteil der detektierbaren Einheit ist. Ebenfalls alternativ kann der Rest R als solches die detektierbare Einheit oder funktionelle Gruppe darstellen.

Derartige markierte Oligonukleotide sind für eine Vielzahl von verschiedenen Anwendungen in der Molekularbiologie, beispielsweise in der Real Time PCR in vorteilhafter Weise zu verwenden. Vorzugsweise handelt es sich bei der detektierbaren Markierung deshalb um einen Fluoreszenzfarbstoff oder ein Fluoreszenzquenchermolekül. Entsprechende Farbstoffe bzw. Moleküle, welche als detektierbare Einheit von Oligonukleotiden dienen können, sind dem Fachmann hinreichend bekannt. Als den Schutzumfang der vorliegenden Erfindung nicht einschränkende Beispiele sind hier zu nennen: Fluoreszeine, Rhodamine, Cyanine, Merocyanine, Carbocyanine sowie Azo- und Poly-Azo-Verbindungen

Gegenstand der vorliegenden Erfindung sind auch Real Time PCR-Sonden der oben beschriebener Struktur, bei denen mindestens eine Fluoreszenzmarkierung über eine Amid-/Elektronenakzeptor-Gruppe an das Phosphatatom der Oligonukleotidkette gebunden ist. Beispiele für derartige Sonden sind FRET-Hybridization Probes (WO 97/46707) oder so genannte Single-labeled Probes (WO 02/14555). Besonders bevorzugt sind in dem Zusammenhang Oligonukleotidsonden, bei denen eine erfindungsgemäße Modifikation intern an einem Internukleosidphosphatrest besteht.

Die vorliegende Erfindung betrifft dabei insbesondere auch dual markierte Oligonukleotide, die zwei detektierbare Einheiten besitzen. Beispiele für derartige Sonden sind TaqMan-Sonden (US 5,804,375) und Molecular Beacons (US 5,118,801). In diesem Zusamenhang betrifft die vorliegende Erfindung dual markierte Oligounkleotide, bei denen eine erste Fluoreszenzmarkierung über eine Amid-/Elektronenakzeptor-Gruppe an ein Inter-Nukleosid-Phosphatatom der Oligonukleotidkette gebunden ist und eine zweite detektierbare Einheit terminal am 5' Ende oder 3' Ende des Oligonukleotids vorhanden ist. Moleküle, die derartige Markierungen aufweisen sowie die Möglichkeiten zu deren Herstellung sind der Fachwelt bekannt.

### Herstellung der erfindungsgemäßen Oligonukleotide

Die vorliegende Erfindung betrifft auch Verfahren zur Herstellung von modifizierten Oligonukleotiden und insbesondere Verfahren zur Herstellung von Oligonukleotiden wie oben beschrieben wurden.

In allgmeiner Form betrifft die vorliegende Erfindung Verfahren zur Herstellung von modifizierten Oligonukleotiden, welche dadurch gekennzeichnet sind, dass ein 3-wertiges Phosphorderivat der chemischen Struktur 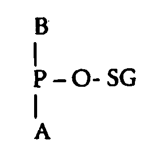 wobei SG eine Schutzgruppe darstellt,
wobei A das 5' Ende eines Nukleotids oder einer Nukleotidkette darstellt, und
wobei B das 3' Ende eines Nukleotids oder einer Nukleotidkette darstellt,
umgesetzt wird mit einem Azid der Struktur

N=N=N-Acc

wobei Acc ein Elektronenakzeptor oder ein mit einem Rest R substituierter Elektronenakzeptor ist und R ein beliebiger organischer Substituent ist.

Als Schutzggruppen werden insbesondere beta-Cyanoethyl, Methyl, Allyl oder Silyl bevorzugt. Alternativ können gemäß der vorliegenden Erfindung auch Methyl-Phosphonate hergestellt werden, bei denen -O -SG durch CH₃ ersetzt ist.

Erfindungsgemäß enthalten die Azide beliebige Elektronenakzeptorgruppen. Diese Gruppen werden dann mit dem jeweiligen Phosphoratom verknüpft. Erfindungsgemäß werden folgende Elektronenakzeptorgruppen verwendet:
a) - CN
b) - SO2-R,
c) sechsgliedrigen N+ - Heterozyklen, ausgewählt aus einer Gruppe bestehend aus Pyridinium, Pyrimidinium und Chinolinium.

Das erfindungsgemäße Verfahren kann insbesondere auch im Rahmen einer konventionellen Oligonukleotidsynthese routinemäßig angewendet werden. Gegenstand der vorliegenden Erfindung ist somit auch ein Verfahren, enthaltend die folgenden Schritte
a) Umsetzung eines 3' Phosphoramidits mit dem 5' -OH Ende einer naszierenden Oligonukleotidkette
b) Umsetzung mit einem Azid der Struktur

   N=N=N-Acc,

   wobei Acc ein Elektronenakzeptor oder ein mit einem Rest R substituierter Elektronenakzeptor ist und R ein beliebiger organischer Substituent ist.

Dabei kann das 5' OH Ende der naszierenden Oligonukleotidkette entweder das 5' Ende eines 5' terminalen Nukleotids oder die freie OH Gruppe eines CPGs darstellen.

Die konventionelle Oligonukleotidchemie beginnt an einem reaktivem Festphasen-Trägermaterial. Als Festphasen-Trägermaterial werden polymere Substanzen bezeichnet, die eine Festphase mit einer reaktiven Gruppe bilden, an denen weitere Moleküle immobilisiert werden können. In der Regel handelt es sich bei der Oligonukleotidsynthese um poröse Glasbeads mit einer definierten Porengröße, sogenannte Contolled Pore Glass Particles (CPG). Alternativ dazu können auch Polystyrolharze und andere organische Polymere und Copolymere verwendet werden. (J. Indian. Chem. Soc. 1998, 75, 206-218) Für den Fall, dass die Oligonukleotide nach der Synthese am Substrat immobilisiert bleiben sollen, können als Festphasen-Trägermaterial Glas oder auch Halbleiterchips verwendet werden. Derartiges Festphasen-Trägermaterial ist kommerziell erhältlich.

Der Träger kann über eine so genannte Verbindungsgruppe enthaltend eine abspaltbare Bindung mit dem einer durch eine Schutzgruppe wie bspw DMT (Dimethoxy-Trityl) geschützten endständigern reaktiven Hdroxylrest verbunden sein. Als Verbindungsgruppe mit abspaltbarer Bindung werden solche Gruppen bezeichnet, die sich zwischen dem trifunktionellen Abstandshalter und dem Festphasen-Trägermaterial befinden und durch eine einfache chemische Reaktion gespalten werden können. Dabei kann es sich um Succinyl- oder Oxalyl- oder andere Verbindungsgruppen handeln, die eine spaltbare Esterbindung enthalten. Weitere Verbindungsgruppen sind dem Fachmann bekannt (J. Indian. Chem. Soc. 1998, 75, 206-218).

Derartige Verbindungsgruppen sind essentiell für die Verwendung des Trägermaterials zur Synthese von Oligonukleotiden, die nach Beendigung der Synthese in wässriger Lösung vorliegen sollen. Für den Fall, dass wie bei der Herstellung von Nukleinsäure-Arrays (US 5,624,711, Shchepinov, M.S., et al., Nucl. Acids. Res. 25 (1997) 1155-1161) das Oligonukleotid nach der Synthese an der Oberfläche des Trägermaterials verbleiben soll, ist dagegen keine spaltbare Verbindungsgruppe erforderlich, sondern es wird vielmehr eine nicht spaltbare Verbindungsgruppe bevorzugt.

Im Einzelnen verläuft eine Oligonukleotidsynthese zum Einbau der erfindungsgemäßen Strukturen wie folgt:

Nach Entfernung der DMT Schutzgruppe durch Säurebehandlung entsteht eine reaktive Hydroxylgruppe, an die eine Kettenverlängerung in Richtung 3'-5' erfolgen kann. In einem zyklischen Verfahren werden dann nacheinander am 5'-Ende ebenfalls mit einer DMT-Schutzgruppe versehene 3'-Phosphoramidit-Derivate von (Desoxy-)-Ribonukleosiden in Gegenwart von Tetrazol an die jeweils von der DMT-Schutzgruppe befreite reaktive Gruppe gekoppelt. Dabei entsteht als Zwischenprodukt ein Intermediat mit einem 3-wertiges Phosphoratom, welches jeweils eine Esterbindung zu den durch die Reaktion miteinander verbundenen Nukeleosiden und eine dritte Esterbindung zu einer geschützten Hydroxylgruppe ausbildet, die dem bereits im eingesetzten Phosphoramidit enthalten ist. Diese Schutzgruppe, die beispielsweise durch beta-Cyanoethyl, Methyl, Allyl oder Silyl gebildet werden kann, wird anschließend-. nach Beendigung der Oligonukleotidsynthese mit Ammoniak abgespalten, wobei auch die Basenschutzgruppen und der Linker zum CPG gespalten wird

An den Positionen, an denen Phosphatmimetika in die Nukleotidkette eingeführt werden sollen, wird anstelle der Oxidation mithilfe von Jod das naszierende Oligonukleotid erfindungsgemäß mit einem Azid der Struktur

N=N=N-Acc

umgesetzt, wobei Acc ein Elektronenakzeptor oder ein mit einem Rest R substituierter Elektronenakzeptor ist und R ein beliebiger organischer Rest ist. Dabei erlaubt die beschriebene Synthese-Chemie den Einbau von prinzipiell beliebigen Resten R und insbesondere den Einbau von jeglicher Art von Fluoreszenzfarbstoffen.

Die Herstellung von Acc-Aziden wie z.b. Acylaziden und Sulfonylaziden ist einfach und seit langem bekannt. (Review: Bräse, S., et al., Angewandte Chemie 117 (2005) 5320-5374, 3.4 und 3.5.2) Sie werden bevorzugt aus den Acyl- oder Sulfonylchloriden mit Natriumaziden oder aus den Hydraziden mit Salpetriger Säure hergestellt.

Farbstoffsulfonylazide werden z.B. auch in Färbeprozessen verwendet (z.B. DE 19650252). Cyanazid kann einfach durch Umsetzung von Natriumazid mit Bromcyan in Acetonitril erzeugt werden (McMurry, J.E., et al., J. Organic Chemistry 38(16) (1973) 2821-7). Heteroarylazide sind durch nukleophile Substitution eines Halogens mit Azid oder aus Heteroarylhydrazinen zugänglich. Bedingung ist, dass der elektronenziehende Stickstoff in para oder ortho Stellung zur Azidogruppe ist, da nur dann ein resonanzstabilisiertes Phosphatmimetikum entsteht. In diesem Zusammenhang besonders geeignet sind ortho oder para N-Alkyl Pyridiniumazide. Acyl-, Sulfonyl- und Pyridylazide sind teilweise auch kommerziell erhältlich.

Gegenstand der vorliegenden Erfindung sind darüber hinaus auch solche Verfahren wie oben beschrieben, bei denen der Rest R eine detektierbare Einheit ist. Vorzugsweise handelt es sich bei R um einen Fluoreszenzfarbstoff oder um ein Fluoreszenz-Quencher Molekül.

Bestimmte Ausführungsformen der vorliegenden Erfindung betreffen die Herstellung von dual markierten Oligonukeotidsonden, bei denen eine Markierung vorzugsweise intern gemäß dem erfindungsgemäßen Verfahren in das Oligonukleotid eingeführt wird und eine weitere Markierung nach aus dem Stand der Technik bekannten Methode vorzugsweise am 5' oder 3' Ende in das Oligonukleotid eingeführt wird.

Bei einer 5'-Markierung an der 5'-Position der Ribose des 5'-terminalen Nukleotids erfolgt der Einbau nach konventionellen Methoden mithilfe eines Farbstoffmarkierten Phosphoramidits am Ende der Oligonukleotid-Synthese (Beaucage, S.L., Methods in Molecular Biology 20 (1993) 33-61, ed. S. Agrawal).

Eine Markierung am 3'-Ende erfolgt dadurch, dass als reaktiver Festphasenträger kommerziell erhältliches CPG verwendet wird, das neben der tritylierten Hydroxylfunktion bereits eine detektierbare Markierung enthält. Nach der Abspaltung der DMT Schutzgruppe kann an der nun freien Hydroxylgruppe die Standard-Oligonukleotidsynthese begonnen werden.

Alternativ können für eine weitere 5' oder 3' Markierung aus dem Stand der Technik bekannte Methoden zum "Post-Labeling" verwendet werden (US 5,002,885, US 5,401,837).

Gegenstand der Erfindung sind auch erfindungsgemäße Syntheseintermediate, die vor der Standard-Oligonukleotidsynthese präpariert werden können. Bevorzugt sind hier noch an der Festphase gebundene noch nicht entschützte Intermediate, die abasische Spacer-Gruppen enthalten können. Zur Herstellung werden bevorzugt CPGs benutzt, die dem Fachmann als Phosphat-CPG geläufig sind, da nach der Oligosynthese ein 3'phosphoryliertes Oligonukleotid entsteht. Solche Phosphat-CPGs werden nach Detritylierung im Gegenwart eines Aktivators mit einem Spacerphosphoramidit umgesetzt: Das gebildete dreiwertige Phosphorintermediat wird dann mit einem Acc-Azid, das eine detektierbare Einheit umfaßt, umgesetzt. Diese Syntheseintermediate können gelagert werden und wie trifunktionelle CPGs zum universellen 3' Labeling eingesetzt werden.

Bestandteil der vorliegenden Erfindung ist auch eine Synthese von Phosphoramiditen, die anstelle eines z.b Beta-Cyanoethyl geschützen Sauerstoffs eine geschützte N-Acc Gruppe enthalten, um die Synthese von N-Acc Phosphorthioaten oder bis-N-Acc Phosphatmimetika ermöglichen. Eine derartige Synthesestrategie eignet sich in Einzelfällen z.Bsp. zur Herstellung von P=N-CN enthaltenden Oligonucleotiden.

Auch bei der Synthese von Methylphosphonaten entsteht eine dreiwertige Phosphorzwischenstufe, die mit Aziden umgesetzt werden können. Methylphosphoramidite sind ebenfalls kommerziell erhältlich.

Bei einer inversen Synthesestrategie (EP 1 155 027) die sowohl für Standardoligonukleotide, als auch insbesondere für Analoga, z.B für die Synthese von N3'->P5'-Olignukleotiden eingesetzt wird, entsteht ebenfalls eine Zwischenstufe enthaltend eine dreiwertigen Phosphor, der erfindungsgemäß mit Aziden umgesetzt werden kann. Die entsprechenden Phosphoramidite sind kommerziell erhältlich.

### Anwendungsspektrum:

Die erfindungsgemäße Synthesestrategie erlaubt die Herstellung unterschiedlichster am Phosphatbackbone modifizierte Oligonukleotide. Der Modifizierungsgrad, die Diversität und die Ladung der Modifikationen werden durch die beabsichtige Anwendung bestimmt.

Beispielsweise können die erfindungsgemäßen Oligonukleotide die zur Hybridisierung mit natürlicher DNA und RNA wie z.B. zum Capturing oder zur Detektion verwendet werden: Oligonukleotide mit P-N=Acc Phosphatmimetika allein oder als Chimäre mit normaler Phosphaten können aber auch erfolgreich als Primer in Amplifikationsreaktionen eingesetzt werden.

Derartige Oligonukleotidsonden sind Basis für verschiedenste Anwendungen z.B Real Time PCR, FISH, Blot Techniken, Sequencing (P.M Jung et al in Nucleic Acid Amplification Technologies BioTechniques Books, Div. Eaton Publishing 1997 Herausg. H.H.Lee, S.A Morse, O. Olsvik, Kapitel 10 Bustin, Stephen A. and Nolan, Tania. Chemistries. IUL Biotechnology Series (2004), 5(A-Z of Quantitative PCR), 215-278.)

Besonders geeignet sind die erfindungsgemäß markierten Oligonukleotide als Fluoreszenz-markierte Sonden in verschiedenen Real Time PCR Formaten:

Für das Molecular Beacon Format (US 5,118,801) sowie für das TaqMan Probe Format (US 5,210,015, US 5,538,848, and US 5,487,972, US 5,804,375) werden in der Regel dual markierte Sonden verwendet, wobei sich in der Regel eine Markierung vorzugsweise intern eingeführt wird und eine zweite Markierung am 5' oder 3' Ende der Sonde befindet. Dabei ist es insbesondere von Vorteil, zumindest die interne Markierung mit einem erfindungsgemäßen Verfahren im Rahmen einer auf der Phosphoramidit-Chemie beruhenden Oligonukleotidsynthese durchzuführen. Die zweite detektierbare Einheit am 5' oder 3' Ende der Sonde kann entweder ebenfalls gemäß einem der beschriebenen erfindungsgemäßen Verfahren oder mithilfe von aus dem Stand der Technik bekannten Verfahren in die entsprechende Sonde eingeführt werden.

Für das FRET Hybridization Probe Format (Matthews, J.A., und Kricka, L.J., Analytical Biochemistry 169 (1988) 1-25), (Bernard, P.S., et al., Analytical Biochemistry 255 (1998) 101-107) werden in der Regel eine 5' terminal und eine 3' terminal markierte Probe verwendet. Dabei ist es insbesondere von Vorteil, die 5' markierte Sonde mit einem erfindungsgemäßen Verfahren im Rahmen einer auf der Phosphoramidit-Chemie beruhenden Oligonukleotidsynthese durchzuführen.

Gemäß der vorliegenden Erfindung lassen sich in einfacher Weise funktionalisierte Oligonukleotide herstellen, indem der Elektronenakzeptor Acc mit einem Rest R modifiziert ist, der eine funktionelle Gruppe, die für die Oligosynthese entsprechend geschützt wurde, enthält. Ist dieser Rest eine Amino- oder Hydroxylaminogruppe, so lassen sich mit solchen z. B. Oligonukleotide Arrays durch spotting auf epoxymodifizierte Oberflächen herstellen (Seliger, H., et al., Current Pharmaceutical Biotechnology, 4 (2003) 379-395). Thiolgruppen können dagegen zur Immobilisierung auf Goldoberflächen oder Goldpartikeln benutzt werden. Hier ist es erfindungsgemäß besonders vorteilhaft, wenn auf einfache Weise mehrere Thiolfunktionen eingeführt werden, um eine stabile Bindung der Fangsonde auf der Goldoberfläche zu erhalten. Wird als funktionelle Gruppe eine geschützte OH Gruppe eingebaut, so können auch verzweigte oder dendritische Oligonukleotide hergestellt werden.

Solche funktionellen Gruppen, insbesondere Aminogruppen, können auch zur Herstellung von markierten Oligonukleotiden verwendet werden, in dem sie nach der Oligosynthese mit dem Aktivesters eines Farbstoffes umgesetzt werden. Vorteilhafter ist es jedoch, entsprechend des erfindungsgemäßen Verfahrens die detektierbare Einheit direkt während der Oligonukleotidsynthese einzuführen.

Da die erfindungsgemäßen Oligonukleotide Nuklease-resistent sind, sind diese auch zum Einsatz im Rahmen von verschiedenen der Fachwelt bekanten Verfahren zur Inaktivierung von Genexpression geeignet, dh die erfindungsgemäßen Oligonukeotide werden als antisense Oligonukleotide oder auch als Bestandteil von siRNA Wirkstoffen eingesetzt. Hierbei können die Modifikationen so gewählt werden, dass die zelluläre Aufnahme erleichtert und/oder die Bindung an die Ziel-Nukleinsäure verbessert wird.

Darüber hinaus können erfindungsgemäße Oligonukleotide als hydrophiler Linker zwischen einer detektierbaren Einheit und einem Protein oder auch als Label mit definierter Masse benutzt werden. Weiterhin können Aptamer Substanzbibliotheken aufgebaut werden, wobei bei der Synthese durch Verwendung von unterschiedlichen Sulfonyl- und Acylaziden oder Heteroarylaziden am Phosphat unterschiedlichste Reste R eingeführt werden können. Solche Bibliotheken können dann hinsichtlich Bindung an Proteine oder andere Biomoleküle getestet werden. Der Vorteil gegenüber aus dem Stand der Technik bekannten Aptameren besteht darin, dass mit dem erfindungsgemäßen Verfahren eine Vielzahl von verschiedenen zusätzlichen Modifikationen auf eine einfache Art und Weise hergestellt und getestet werden kann.

### Beispiele

### Beispiel 1

### Synthese eines modifizierten dT(P(=NSO2PhNHAc)dT

Die Dimer Synthese wurde im 10 µmol Scale an einem ABI 394 Synthesizer durchgeführt. Kommerziell erhältliches dT CPG Support wurde als Festphase benutzt. Alle Chemikalien für die Standardsynthese wurden von Glen Research erworben.

Die konventionelle Oxidizer Lösung enthaltend Iod wurde durch eine 0.1 M Lösung von p-NAc Phenylsulfonsäureazid (Sigma Aldrich) in wasserfreien Acetonitril ersetzt. Die Oxidätionszeit wurde auf 16 min verlängert.

Das Produkt wurde mit 33 % Ammoniak für 2h bei Raumtemperatur vom Träger gespalten und mittels Reversed Phase Chromatographie an einer Poros Oligo R3 4.6 x 50 mm Säule getrennt. Chromatographie: Puffer A: 0.1M Triethylammoniumacetat in Wasser pH 6.8 Buffer B: 0.1 M Triethylammoniumacetat in Wasser/Acetonitril 1:1; Gradient 2 min 0 % B in 45 min auf 100 %B. Die UV Absorption des Eluenten wird bei 260 nm gemessen. Es ergab sich eine Hauptfraktion, die das gewünschte Produkt enthielt. Die Lösungsmittel wurden an einer Vakuum Zentrifuge entfernt. Der Rückstand wurde in bidest. Wasser aufgenommen und es wurde wieder im Vakuum evaporiert. Dieses Prozedere wurde dreimal wiederholt. Der Rückstand wurde dann in bidest. Wasser gelöst und lyophilisiert.

1H NMR: ( Bruker DPX 300) in D20: 7.82 d [2H, Aryl], 7.56 d[2H Aryl] 7.47 s[ 1H, C6-H], 7.40[1H, C6-H], 6.21 m [1 H, H1'], 6.21 m [1 H, H1'], 6.07 m[1 H, H1'], 4.38 m [1H, H 3'],
4.10 [m 4 H, H4', H5'] 2.38- 2.24 m [4 H, H2'] 2.22 [3 H, CH3] 2.16[3 H, CH3], 2.14 [3 H, CH3]
31P NMR: (Bruker DPX 300) in D2O: 2.14
Massen Spektroskopie( ESI-MS) calc 742.66 found [M-H]: 741.73

### Beispiel 2

### Synthese eines T(P(=NSO2PhNHAc) T9 Oligonucleotides

Die Oligonucleotide Synthese wurde im 1 µmol scale an einem ABI 394 Synthesizer durchgeführt. Kommerziell erhältliches dT CPG support wurde als Festphase benutzt. Alle Chemikalien für die Standardsynthese wurden von Glen Research erworben.

Im ersten Synthese Zyklus wurde der Oxidizer enthaltend Iod durch eine 0.1 M Lösung von p-NAc Phenylsulfonsäureazid (Sigma Aldrich) in wasserfreien Acetonitril ersetzt. Die Oxidationszeit wurde auf 16 min verlängert. Die Anknüpfung der restlichen dT-Phosphoramidite wurde entsprechend der Standardprotokolle durchgeführt.

Das Produkt wurde mit 33 % Ammoniak für 2h bei Raumtemperatur vom Träger gespalten und mittels Reversed Phase Chromatographie an einer Poros Oligo R3 4.6 x 50 mm Säule getrennt. Chromatographie: Puffer A: 0.1M Triethylammoniumacetat in Wasser pH 6.8 Buffer B: 0.1 M Triethylammoniumacetat in Wasser/Acetonitril 1:1; Gradient 2 min 0 % B in 45 min auf 100 %B. Die UV Absorption des Eluenten wurd bei 260 nm gemessen. Es ergab sich eine Hauptfraktion, die das gewünschte Produkt enthielt. Die Lösungsmittel wurden an einer Vakuum Zentrifuge entfernt. Der Rückstand wurde in bidest. Wasser aufgenommen und es wurde wieder im Vakuum evaporiert.

Dieses Prozedere wurde dreimal wiederholt. Der Rückstand wurde dann in bidest. Wasser gelöst und lyophilisiert
Massen Spektroskopie ( ESI-MS) calc 3176.25 found [M-H]: 3176.0

### Beispiel 3

### Synthese eines Fluorescein-markierten Oligonukeotids

### 5' AAT ACC TGT ATT CCT CGC CTG TC Fluorescein-3' wobei jedes P=O durch P=N-pPh-NAc ersetzt ist

Die Oligonucleotide Synthese wurde im 1 µmol scale an einem ABI 394 Synthesizer durchgeführt. Kommerziell erhältliches LightCycler Fluorescein CPG (Roche Applied Science) wurde als Trägermaterial benutzt. Alle Chemikalien für die Standardsynthese wurden von Glen Research erworben. Phosphoramidite mit tert butylphenoxyacetyl Schutzgruppen (bekannt als "tac" oder "Expedite" Monomere) von Proligo wurden verwendet.

Für die Synthese wurde das Standardprotokol verwendetet,wobei der Oxidizer enthaltend Iod durch eine 0.1 M Lösung von p-NAc Phenylsulfonsöureazidazid (Sigma Aldrich) in wasserfreien Acetonitril ersetzt und die Oxidationszeit auf 16 min verlängert wurde.

Das Produkt wurde mit 33 % Ammoniak für 2h bei Raumtemperatur vom Träger gespalten und mittels Reversed Phase Chromatographie an einer Poros Oligo R3 4.6 x 50 mm Säule getrennt. Chromatographie: Puffer A: 0.1M Triethylammoniumacetat in Wasser pH 6.8 Buffer B: 0.1 M Triethylammoniumacetat in Wasser/Acetonitril 1:1; Gradient 2 min 0 % B in 45 min auf 100 %B. Die UV Absorption des Eluenten wurde bei 260 nm gemessen. Es ergab sich eine Hauptfraktion, die das gewünschte Produkt enthielt. Die Lösungsmittel wurden an einer Vakuum Zentrifuge entfernt. Der Rückstand wurde in bidest. Wasser aufgenommen und es wurde wieder im Vakuum evaporiert. Dieses Prozedere wurd dreimal wiederholt. Der Rückstand wurde dann in bidest. Wasser gelöst und lyophilisiert.

Massen Spektroskopie (ESI-MS) calc: 11839 found [M-H]: 11839.9

### Beispiel 4

### a) Herstellung von Dabsylazid

0.71 g (2.19 mmol) Dabsylchlorid wurden in 10 ml Aceton gelöst. Unter Eiskühlung und Rühren wurde eine Lösung von 142 mg (2.19 mmol) Natriumazid in 2 ml Wasser langsam zugetropft. Es wurde 2 h bei 0°C und dann 2 h bei Raumtemperatur gerührt. Dann wurde eine Lösung von 32 mg Natriumazid (0.5 mmol) in 500 µl Wasser zugegeben und 1 h bei Raumtemp.eratur gerührt. ( DC Kieselgel CH2Cl2). Die Suspension wurde sodann mit 200 ml Methylenchlorid versetzt und filtriert. Das Filtrat wurde 2 mal mit Wasser einmal mit 5 % Natriumhydrogencarbonat Lösung und dann zweimal mit Wasser ausgeschüttelt, Die abgetrennte organische Phase wurde über Natriumsulfat getrocknet. Das Lösungsmittel wurde am Rotationsverdampfer bei einer Badtemperatur von < 20 °C abdestilliert. Der Rückstand wird in 2 ml Acetonitril suspendiert und abfiltriert. Dieser Rückstand wurde mit Ether gewaschen.

Rohausbeute 280 mg. Das Azid wird ohne weitere Reinigung direkt am DNA-Synthesizer oder zur Herstellung eines Trägers benutzt.

### b) Synthese chimärer Oligonukleotides bei denen an gezielten Positionen P=O durch P=N-Acc ersetzt wurde

Die Synthesen wurden im 1 mol scale an einem ABI 394 Synthesizer durchgeführt. Um nicht während der Synthese den Oxidizer wechseln zu müssen, wurde das Syntheseprogramm so modifiziert, dass die N3-Acc Lösung an eine extra Basenposition angeschraubt werden kann. Aufgrund von Limitationen der Programmierung wurde das Azid zusammen mit Aktivator umgesetzt. Dies hatte keinen Einfluß auf die Modifikation. Die Reaktionszeit des N3 Acc mit dem 3 wertigen Phosphorintermediat war 5 min.

Alle Chemikalien für die Standardsynthese wurden von Glen Research erworben. Phosphoramidite mit tert butylphenoxyacetyl Schutzgruppen (bekannt als "tac" oder "Expedite" Monomere) von Proligo wurden verwendet. Die Reinigung erfolgte wie oben beschrieben.

Träger: Fluorescein CPG: N3-Acc: p-NAc Phenylsulfonsäureazid(Sigma Aldrich)

Folgende Sonden mit jeweils gleicher SEQ. ID. NO: 4 wurden synthetisiert und anschließend massenspektroskopisch analysiert:

| Modifikation (SEQ: ID. No: 4) | Masse berechnet | Masse gemessen |
|---|---|---|
| 5' AATACCTGTATTCCTCGCCTGTplCFluorescein-3' | 7718 | 7718.6 |
| 5' AplATACCTGTATTCCTCGCCTGTC Fluorescein-3' | 7718 | 7718.9 |
| 5' AATACCTGp1TATTCCTCp1GCCTGTC-Fluorescein-3' | 7915 | 7914.9 |
| 5' Ap1Ap1Tp1Ap1Cp1Cp1Tp1Gp1Tp1Ap1Tp1TCCTCGCCTGTC-Fluorescein-3' | 9681 | 9681.9 |
| 5' AATACCTGTATrp1Cp1Cp1Tpl1Cp1Gpl1Cp1Cp1Tp1Gp1Tp1C-Fluorescein-3' | 9681 | 9681.8 |
| 5'Ap1Ap1Tp1ACCTGTATp1Tp1Cp1CTCGCCTp1Gp1Tp1C-Fluorescein-3' | 9288 | 9289.8 |

| | | |
|---|---|---|
| p1 ist ein P=N-pPh-NAc Mimetikum, | | |

### Referenz - Beispiel 5

### Erfindungsgemäße 3'-terminale Markierung

### a) Herstellung eines Dabsyl Trägers für die Oligonukleotidynthese

### CPG- Icaa- NHC(=O)-CH₂CH₂- C(=O)-O-CH₂-CH₂-SO₂-CH₂-CH₂-O-P(O-CH₂-CH₂-CN)(=N-SO₂-Ph-p-N=N-Ph-p-NMe₂)-O CH₂-CH₂-CH₂- ODMTr

1.2 g Phospholink CPG Load 49 µmol/g wurde in eine Schlenkfritte gefüllt und unter Argon mit wasserfreien Acetonitril gewaschen. Dann wurde mit 0.1M Dichloressigsäure in Methylenchlorid solange gewaschen bis das Filtrat farblos ist. Anschließend wurde gründlich mit wasserfreien Acetonitril gewaschen. Anschließend wurde mit 2 ml 0.25 M Dicyanoimidazol in Acetonitril (Aktivator) gewaschen und 2 ml Aktivator und sofort 2 ml 0.1 M Lösung des Spacer C3 Phosphoramidites zugegeben. Die Suspension wurde danach 3 min stehen gelassen. Unter Argondruck wurde filtriert. Dann wurde mit 2 ml Aktivator gewaschen und wiederholt 2 ml Aktivator und sofort 2 ml 0.1 M Lösung des Spacer C3 Phosphoramidites zugegeben. Danach wurde der Ansatz 12 min stehengelassen. Unter Argondruck wurden die Lösungsmittel abfiltriert, 2 ml einer 0.1 M von Dabsylazid in Methylenchlorid zugegeben und die Mischung wird 15 min stehengelassen. Das modifizierte CPG wurde schließlich mit 100 ml Methylenchlorid dann mit 100 ml wasserfreien Acetonitril gewaschen und im Vakuum getrocknet.

### b) Oligonukleotidsynthese unter Verwendung des Dabsylträgers:

### 5' Fluorecein-GCA CCA GAT CCA CGC CCT TGA TGA GC-O CH₂-CH₂-CH₂- O (O₂)P(=N-SO₂-Ph-p-N=N-Ph-p-NMe₂)

Die Oligonukleotide Synthese wurde im 1 µmol scale an einem ABI 394 Synthesizer durchgeführt. Das Dabsyl-CPG aus Bsp 5a wurde als Trägermaterial benutzt. Zum 5'Labeling wurde 6-Carboxyfluorescein Phosphoramidite (Glen Research, Report No. 10 (GR10-1) (1997) 1-12) benutzt.

Alle anderen Chemikalien für die Standardsynthese wurden von Glen Research erworben. Wie unter 4 b) beschreiben wurden Phosphoramidite mit tert butylphenoxyacetyl Schutzgruppen (bekannt als "tac" oder "Expedite" Monomere) von Proligo verwendet. Die Synthese wurde nach Standardprotokoll durchgeführt verwendetet. Die Abspaltung und Aufreinigung erfolgte ebenfalls wie in 4b beschrieben.

Massen Spektroskopie (ESI-MS) calc: 8973 found [M-H]: 8973.1

### Beispiel 6

### Real Time PCR und Schmelzkurvenanalytik

Um den Einfluß von Phosphatmimitika auf die Hybridisierung zu analysieren, wurde eine quantitative Real time PCR von Factor V DNA mit anschließender Schmelzkurvenanlytik am LightCycler 1.2 ^{®} Instrument (Roche Diagnositcs GmbH) durchgeführt. Es wurden Primer in Kombination mit einem Paar aus Fluorescein/ LightCycler Red 640 FRET Hybridisieungssonden eingesetzt. Die Primer und die 5'LightCycler Red 640 Sonde wurde konstant gehalten. Als FRET Donor Proben wurden die unterschiedlich am Phosphat modifzierte 3'Fluorescein-Sonden aus Beispiele 4 bzw als Referenz die unmodizierte Fluorescein Sonde eingesetzt. Es wurde der Einfluß auf den crossing point, der ein Maß für die Amplifikationseffizienz ist und auf den Schmelzpunkt bewertet.

Für die Anmplifikation eines Factor V DNA Fragment wurden 20µl einer PCR Reaktonsmischung wie folgt angesetzt.

| | |
|---|---|
| 10⁶ | Kopien eines Plasmids, das das Factor V Gen Wildtyp und Mutante beinhaltet (Gene Bank Accession No: M_014335 ) |
| | |
| 13 mM | MgCl2 |
| | |
| 500 nM each | Primer mit den SEQ. ID. NO: 1 und 2 |
| | |
| 200 nM each | FRET Hybridization Sonden mit den SEQ.ID. NO: 3 und 4 |

Für alle weiteren PCR Komponenten wurde ders LightCycler DNA Master Hyb Probes Kit (Roche Applied Science, Cat. No. 2158825) gemäß den Angaben des Herstellers verwendet.

Als Primer und Sonden wurden folgende Sequenzen verwendet:
SEQ ID NO:1
   Forward Primer:
   5' GAG AGA CAT CGC CTC TGG GCT A
SEQ ID NO:2
   Reverse Primer
   5'TGT TAT CAC ACT GGT GCT AA
SEQ ID NO:3
   FRET Akzeptor Sonde
   5' LC-Red 640 AGG GAT CTG CTC TTA CAG ATT AGA AGT AGT CCT ATT
SEQ ID NO:4
   FRET Donor Sonde
   5' AAT ACC TGT ATT CCT CGC CTG TC-Fluorescein

Für die Amplifikation am LightCycler 1.2 (Roche Applied Science) wurde folgendes Temperaturprogramm benutzt.

| | T[°C] | t[sec] | Ramp-rate[°C/sec] | Acquisition | Cycles |
|---|---|---|---|---|---|
| | | | | | |
| Denaturation | 95 | 30 | 20.0 | none | 1 |
| | | | | | |
| Amplification | 95 | 0 | 20.0 | none | |
| | | | | | |
| | 55 | 10 | 20.0 | single | 45 |
| | | | | | |
| | 72 | 10 | 20.0 | none | |

Das Real time Monitoring wurde mit der 2nd Derivative Threshold Method über 45 Zyklen durchgeführt, wobei das Fluoreszenz Signal in einem dem Detektions Kanal der für die LightCycler Red 640 Emssion spezifisch ist (bei 640 nm) gemessen und der arithmetic Background Korrektur Modus zur Normalisierung des Anfangssignals benutzt wurde.

Nach der Amplifikation wurde eine Schmelzkurvenanalytik entsprechend der Instruktionen des LightCycler Manual (Roche Applied Sciences) durchgeführt. Folgendes Temperaturprogramm wurde benutzt

| | T[°C] | t[sec] | Ramp-rate[°C/sec] | Acquisition | Cycles |
|---|---|---|---|---|---|
| | | | | | |
| Melting curve | 95 | 0 | 20.0 | none | |
| | | | | | |
| | 45 | 60 | 20.0 | continous | 1 |
| | | | | | |
| | 75 | 10 | 0.1 | none | |
| | | | | | |
| Cooling | 40 | 30 | 20.0 | none | 1 |

Die absolute Fluoreszenzsignale werden wird wie oben im 640nm Kanal gemessen, anscchliessend wurde daraus die erste Ableitung kalkuliert.

In der nachfolgenden Tabelle sind die crossing points als Maß für die Amplifikationseffizienz bei Verwendung von verschiedenen modifizierten Donor-Sonden angegeben. Darüber hinaus sind in der Tabelle die gemessenen Schmelztemperaturen der verschedenen Donor-Sonden für die Faktor V Wildtyp-Sequenz und die Faktor V Mutatnten-Sequenz angegeben.

| Modifikation (SEQ. ID. No: 4) | Cp | Tm wt | Tm mt |
|---|---|---|---|
| 5'AATACCTGTATTCCTCGCCTGTC-Fluorescein-3'(Ref) | 22.11 | 64.92 | 56.98 |
| 5' AATACCTGTATTCCTCGCCTGTp1CFluorescein-3' | 22.61 | 64.32 | 56.28 |
| 5' Ap1ATACCTGTATTCCTCGCCTGTC Fluorescein-3' | 22.51 | 64,80 | 56.80 |
| 5'AATACCTGp1TATTCCTCp1GCCTGTC-Fluorescein-3' | 22.26 | 64.28 | 55.89 |
| 5'Ap1Ap1Tp1Ap1Cp1Cp1Tp1Gp1Tp1Ap1Tp1TCCTCGCCT GTC- Fluorescein-3' | 21.40 | 60.69 | 52.19 |
| 5' AATACCTGTATTp1Cp1Cp1Tp1Cp1Gp1Cp1Cp1Tp1Gp1Tp1 C-Fluorescein-3' | 21.91 | 60.74 | 52.14 |
| Ap1Ap1Tp1ACCTGTATp1Tp1Cp1CTCGCCTp1Gp1Tp1C-Fluorescein-3' | 22.00 | 61.40 | 52.83 |

Wie aus der Tabelle ersichtlich, wird der Crossing Point durch die Einführung der erfindungsgemäßen Modifikationen nicht wesentlich beeinflußt, dh die PCR Effizienz ist unverändert.

Darüber hinaus ist bei den 1-2-fach modifizierten Sonden auch kein Einfluß auf die gemessene Schmelztemperatur festzustellen. Zusätzlich zeigen auch vielfach modifizierte Sonden lediglich eine moderate Schmelzpunktänderung von maximal 4 °C, wobei die Fähigkeit zur Mismatchdiskriminierung erhalten bleibt.

### Beispiel 7

### Real Time PCR + Schmelzkurvenanalytik

### a) Lissaminazid-Herstellung

Bei 0°C wurde eine Lösung von 195 mg (3.0 mmol) Natriumazid in 5 ml Wasser zu einer Lösung von 577 mg (1mmol) Sulforhodamin B Säurechlord in 20 ml Aceton getropft. Die Mischung wurde 2 h bei 0°C und dann 6 h bei Raumtemperatur gerührt. Die Mischung wurde in einen Scheidetrichter überführt und mit 300 ml Wasser und 300 ml Methylenchlorid versetzt. Die organische Phase wurde abgetrennt und 2 mal mit 100 ml Wasser gewaschen. Die org. Phase wurde über Natriumsulfat getrocknet. Das Lösungsmittel wurde am Rotationsverdampfer abdestilliert. Der Rückstand (140 mg) wurde ohne weitere Aufreinigung für die Oligonukleotidsynthese verwendet.

### b) Synthese eine SEQ ID NO:3 FRET Akzeptor-Probe

### Ap*GG GAT CTG CTC TTA CAG ATT AGA AGT AGT CCT ATT-p

### p* = P=N S(O)2-Rhodamin-B

Die Oligonucleotide Synthese wurde im 1 µmol scale an einem ABI 394 Synthesizer durchgeführt. Kommerziell erhältliches Phospholink CPG (Glen Research) wurde als Trägermaterial benutzt. Alle Chemikalien für die Standardsynthese wurden von Glen Research erworben. Phosphoramidite mit tert butylphenoxyacetyl Schutzgruppen (bekannt als "tac" oder "Expedite" Monomere) von Proligo wurden verwendet.

Für die Synthese wurde das Standardprotokol verwendetet (trityl off), wobei der Oxidizer im letzten Zyklus durch eine 0.1 M Lösung von Lissaminazid in wasserfreien Acetonitril ersetzt wurde und die Oxidationszeit auf 16 min verlängert wurde.

Das Produkt wurde mit 33 % Ammoniak für 2 h bei Raumtemperatur vom Träger gespalten und mittels Reversed Phase Chromatographie an einer Poros Oligo R3 4.6 x 50 mm Säule getrennt. Chromatographie: Puffer A: 0.1M Triethylammoniumacetat in Wasser pH 6.8 Buffer B: 0.1 M Triethylammoniumacetat in Wasser/Acetonitril 1:1; Gradient 2 min 0 % B in 45 min auf 100 %B. Die UV Absorption des Eluenten wurde bei 260 nm gemessen. Es ergab sich eine Hauptfraktion, die das gewünschte Produkt enthielt. Die Lösungsmittel wurden an einer Vakuum Zentrifuge entfernt. Der Rückstand wurde in bidest. Wasser aufgenommen und es wurde wieder im Vakuum evaporiert. Dieses Prozedere wurde dreimal wiederholt. Der Rückstand wurde dann in bidest. Wasser gelöst und lyophilisiert. Massen Spektroskopie ( ESI-MS) calc: 11710 found [M-H]: 11710 .5

### c) Real Time PCR

Um die Eignung in der Real Time PCR zu zeigen wurde eine Real Time PCR von Factor V DNA am LightCycler 2.0 durchgeführt. Es wurden Primer in Kombination mit einem Paar aus Fluorescein/ Lissamin FRET Hybridisierungssonden eingesetzt, wobei Lissamin als FRET-Akzeptor fungiert. Es wurden Quantifizierzungskurven aufgenommen und der cp-Wert in Abhängigkeit von der Konzentration der Target-Nukleinsäure ermittelt.

Für die Amplifikation von 10⁴ bzw. 10⁶ Kopien eines Factor V DNA Fragments wurde eine Real Time PCR und Schmelzkurvenanalyse gem. Beispiel 6 durchgeführt. Dabei wurde eine nicht weiter modifizierte Fluorescein Donor Probe gem. Beispiel 6 (SEQ ID NO:4) und eine FRET-Akzeptor Probe gem. Beispiel 7b (SEQ ID NO:3) verwendet.

| | T[°C] | t[sec] | Ramp-rate[°C/sec] | Acquisition | Cycles |
|---|---|---|---|---|---|
| | | | | | |
| Denaturation | 95 | 30 | 20.0 | none | 1 |
| | | | | | |
| Amplification | 95 | 0 | 20.0 | none | |
| | | | | | |
| | 55 | 10 | 20.0 | single | 45 |
| | | | | | |
| | 72 | 10 | 20.0 | none | |

Das Fluoreszenz Signal wurde im 610 nm Detektions Kanal gemessen. Der 610/530-Background Korrektur Modus wurde zur Normalisierung des Rohsignals benutzt. Als cp-Werte wurden für 10⁶ Kopien cp 22 und für 10⁴ Kopien cp 26 bestimmt.

Als Schmelzpunkt wurden für den Wildtyp 64.69 °C und für die Mutante 56.24 °C bestimmt (10⁶ Kopien).

### Referenzliste

Agrawal, S., Methods in Mol. Biology, Vol. 26, Protocols for Oligonucleotide Conjugates, Humana Press, Totowa, NJ, 1993, Chapter 3
Baschang, G., und Kvita, V., Angewandte Chemie 85(1) (1973) 43-44
Beaucage, S.L., Methods in Molecular Biology 20 (1993) 33-61, ed. S. Agrawal
Bernard, P.S., et al., Analytical Biochemistry 255 (1998) 101-107
Bräse, S., et al., Angewandte Chemie 117 (2005) 5320-5374, 3.4 und 3.5.2
Burgers, Peter M.J., und Eckstein, Fritz, Biochemistry 18, (1979) 592-6
DE 19650252
EP 1 155 027
Glen Research, Report No. 10 (GR10-1) (1997) 1-12
Greene T.W. Protective groups in organic synthesis.Wiley Interscience Publications John Wiley&Sons, 1981 New York, Chichester, Brisbane Toronto
Hansch, C., et al., Chem Reviews 91 (1991) 165-195
Hodges, R.R., et al., Biochemistry 28 (1989) 261-7
Indian, J., Chem. Soc. 1998, 75, 206-218
Jung, P.M, et al., Nucleic Acid Amplification Technologies BioTechniques Books, Div. Eaton Publishing 1997 Herausg. H.H.Lee, S.A Morse, O. Olsvik, Kapitel 10 Bustin, Stephen A. and Nolan, Tania. Chemistries. IUL Biotechnology Series (2004), 5(A-Z of Quantitative PCR), 215-278
Matthews, J.A., und Kricka, L.J., Analytical Biochemistry 169 (1988) 1-25
McMurry, J.E., et al., J. Organic Chemistry 38(16) (1973) 2821-7
Miller, P.S., et al., Biochemistry 18 (1979) 5134-43
Nielsen, J., und Caruthers, M.H., J. Am. Chem. Soc. 110 (1988) 6275-6276
Seliger, H., et al., Current Pharmaceutical Biotechnology, 4 (2003) 379-395
Shchepinov, M.S., et al., Nucl. Acids. Res. 25 (1997) 1155-1161
Souveaux, E., Methods in Mol. Biology, Vol 26, Protocols for Oligonucleotide Conjugates, Humana Press, Totowa, NJ, 1994, Chapter 1, ed. S. Agrawal
US 4,997,928
US 5,002,885
US 5,118,801
US 5,210,015
US 5,231,191
US 5,241,060
US 5,260,433
US 5,290,925
US 5,401,837
US 5,487,972
US 5,538,848
US 5,624,711
US 5,656,744
US 5,668,266
US 5,804,375
US 6,130,323
WO 01/14401
WO 02/14555
WO 03/02587
WO 91/08213
WO 97/46707
Wojczewski, C., et al., Synlett 10 (1999) 1667-1678

## Patentansprüche

1. Oligonukleotid, enthaltend mindestens einmal die Struktur 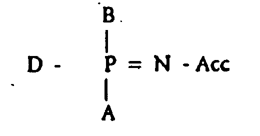 wobei A das 5' Ende eines Nukleotids oder einer Nukleotidkette
B das 3' Ende eines Nukleotids oder einer Nukleotidkette darstellt und
D entweder Hydroxyl oder CH₃ ist
Acc ein Elektronenakzeptor oder ein mit einem Rest R substituierter Elektronenakzeptor ist und R ein beliebiger organischer Substituent ist,
**dadurch gekennzeichnet, dass** Acc ausgewählt wird aus einer Gruppe bestehend aus
- CN,
- SO2 - R', wobei R' mindestens ein Amino-substituiertes Alkyl, ein gegebenenfaUs substituiertes Aryl oder einen gegebenenfalls substituierten Heterozyklus enthält,
- und sechsgliedrigen N+ - Heterozyklen, ausgewählt uns einer Gruppe bestehend aus Pyridinium, Pyrimidinium und Chinolinium.

2. Verfahren zur Herstellung eines modifizierten Oligonukleotids, nach Anspruch 1
**dadurch gekennzeichnet, dass** ein 3-wertiges Phosphorderivat der chemischen Struktur 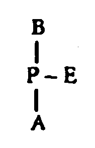 wobei E entweder eine Methylgruppe oder eine geschützte Hydroxylgruppe darstellt,
wobei A das 5' Ende eines Nukleotids oder das 5'Ende einer Nukleotidkette darstellt, und
wobei B das 3' Ende eines Nukleotids oder das 3'Ende einer Nukleotidkette darstellt, umgesetzt wird mit einem Azid der Struktur
N=N=N-Acc
wobei Acc ein Elektronenakzeptor oder ein mit einem Rest R substituierter Elektronenakzeptor ist und R ein beliebiger organischer Substituent ist,
**dadurch gekennzeichnet, dass**
Acc ausgewählt wird aus einer Gruppe bestehend aus
-CN, - SO2 -R
und sechsgliedrigen N+ - Heterozyklen, ausgewählt aus einer Gruppe bestehend aus Pyridinium, Pyrimidinium und Chinolinium.

3. Verfahren nach Anspruch 2, enthaltend die folgenden Schritte
a) Umsetzung eines 3' Phosphoramidits mit dem 5' -OH Ende einer naszierenden Oligonukleotidkette
b) Umsetzung mit einem Azid der Struktur
N = N = N - Acc,
wobei Acc ein Elektronenakzeptor oder ein mit einem Rest R substituierter Elektronenakzeptor ist und R ein beliebiger organischer Substituent ist.

4. Verwendung eines Oligonukleotids gemäß Anspruch 1 als Hybridisations-Partner

5. Verwendung gemäß Anspruch 4 als Hybridisationssonde

## Claims

1. Oligonucleotide containing at least once the structure 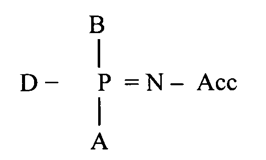 in which A is the 5' end of a nucleotide or of a nucleotide chain
B is the 3' end of a nucleotide or of a nucleotide chain and
D is either hydroxyl or CH₃
Acc is an electron acceptor or an electron acceptor substituted with a residue R and R is any organic substituent,
**characterized in that** Acc is selected from a group comprising
- CN,
- SO₂-R' where R' contains at least one amino-substituted alkyl, an optionally substituted aryl or an optionally substituted heterocycle,
- and six-membered N⁺ heterocycles selected from a group comprising pyridinium, pyrimidinium and quinolinium.

2. Process for producing a modified oligonucleotide according to claim 1, **characterized in that** a trivalent phosphorus derivative of the chemical structure 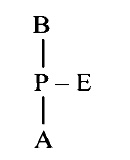 in which E represents either a methyl group or a protected hydroxyl group,
A represents the 5' end of a nucleotide or the 5' end of a nucleotide chain and
B represents the 3' end of a nucleotide or the 3' end of a nucleotide chain
is reacted with an azide of the structure
N=N=N-Acc
in which Acc is an electron acceptor or an electron acceptor substituted with a residue R and R is any organic substituent,
**characterized in that**
Acc is selected from a group comprising
- CN, -SO₂ - R
and a six-membered N⁺-heterocycles selected from a group comprising pyridinium, pyrimidinium and quinolinium.

3. Process according to claim 2, comprising the following steps
a) reacting a 3' phosphoramidite with the 5' OH end of a nascent oligonucleotide chain
b) reaction with an azide of the structure
N=N=N-Acc,
in which Acc is an electron acceptor or an electron acceptor substituted with a residue R and R is any organic substituent.

4. Use of an oligonucleotide according to claim 1 as a hybridization partner.

5. Use according to claim 4 as a hybridization probe.

## Revendications

1. Oligonucléotide contenant au moins une fois la structure 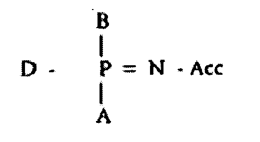 dans laquelle
A représente l'extrémité 5' d'un nucléotide ou d'une chaîne nucléotidique ;
B représente l'extrémité 3' d'un nucléotide ou d'une chaîne nucléotidique ; et
D représente, soit un groupe hydroxyle, soit un groupe CH₃ ;
Acc représente un accepteur d'électrons ou un accepteur d'électrons substitué avec un radical R ; et
R représente n'importe quel substituant organique ;
**caractérisé en ce que** Acc est choisi parmi un groupe constitué par :
- un groupe CN ;
- un groupe SO₂-R' où R' contient au moins un groupe alkyle substitué avec un groupe amino, un groupe aryle éventuellement substitué ou un groupe hétérocyclique éventuellement substitué ; et
- des groupes N+-hétérocycliques à six membres, choisis parmi un groupe constitué par un groupement pyridinium, un groupement pyrimidinium et un groupe quinolinium.

2. Procédé pour la préparation d'un oligonucléotide modifié, selon la revendication 1, **caractérisé en ce qu'**on fait réagir un dérivé de phosphore trivalent possédant la structure chimique 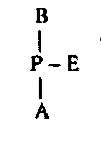 dans laquelle
E représente, soit un groupe méthyle, soit un groupe hydroxyle protégé ;
A représente l'extrémité 5' d'un nucléotide ou l'extrémité 5' d'une chaîne nucléotidique ;
B représente l'extrémité 3' d'un nucléotide ou l'extrémité 3' d'une chaîne nucléotidique ;
avec un azide possédant la structure
N=N=N-Acc
dans laquelle
Acc représente un accepteur d'électrons ou un accepteur d'électrons substitué avec un radical R ; et
R représente n'importe quel substituant organique ;
**caractérisé en ce que** Acc est choisi parmi un groupe constitué par :
- un groupe CN ;
- un groupe SO₂-R ; et
- des groupes N+-hétérocycliques à six membres, choisis parmi un groupe constitué par un groupement pyridinium, un groupement pyrimidinium et un groupe quinolinium.

3. Procédé selon la revendication 2, comprenant les étapes suivantes
a) de mise en réaction d'un 3'-phosphoramidite avec l'extrémité 5'-OH d'une chaîne oligonucléotidique naissante ;
b) de mise en réaction avec un azide possédant la structure
N=N=N-Acc
dans laquelle
Acc représente un accepteur d'électrons ou un accepteur d'électrons substitué avec un radical R ; et
R représente n'importe quel substituant organique.

4. Utilisation d'un oligonucléotide selon la revendication 1, comme partenaire d'hybridation.

5. Utilisation selon la revendication 4, comme sonde d'hybridation.


### SEQUENCE LISTING

<110> Roche Diagnostics GmbH
   F. Hoffmann-La Roche AG
<120> Polynukleotid mit Phosphatmimetikum
<130> 23428
<160> 4
<170> PatentIn version 3.2
<210> 1
   <211> 22
   <212> DNA
   <213> Artificial
<220>
   <223> primer
<400> 1
   gagagacatc gcctctgggc ta 22
<210> 2
   <211> 20
   <212> DNA
   <213> Artificial
<220>
   <223> primer
<400> 2
   tgttatcaca ctggtgctaa 20
<210> 3
   <211> 36
   <212> DNA
   <213> Artificial
<220>
   <223> probe
<400> 3
   agggatctgc tcttacagat tagaagtagt cctatt 36
<210> 4
   <211> 23
   <212> DNA
   <213> Artificial
<220>
   <223> probe
<400> 4
   aatacctgta ttcctcgcct gtc 23